# EUROPEAN PATENT APPLICATION

(11) **EP 1 679 040 A1**
(43) Date of publication of application: **12.07.2006**
(21) Application number: 04789836.6
(22) Date of filing: 15.10.2004
(51) Int. Cl.: A61B 17/00

(54) **EASILY RETRIEVED BIOLOGICAL SPECIMEN POUCH**

(30) Priority: 17.10.2003 CN 200310111814
(71) Applicant: Zhou, Xing, Guangzhou, Guangdong 510060 (CN); Li, Yanfang, Guangzhou, Guangdong 510060 (CN)
(72) Inventor: ZHOU, Xing, Guangzhou, Guangdong 510060 (CN); LI, Yanfang, Guangzhou, Guangdong 510060 (CN); GUO, Aijun, Guangzhou, Guangdong 510060 (CN)
(74) Representative: Stenborg, Anders Vilhelm
(86) International application number: PCT/CN2004/001174
(87) International publication number: WO 2005/037107

(57) **Abstract**

The invention relates to an easily retrieved biological specimen pouch. The easily retrieved biological specimen pouch of the invention comprises flexible wall, open end and closed end, and the specimen pouch can receive a biological specimen; A) The flexible wall of the open end of the specimen pouch has discontinuous serration; B) On the serration, there are slots through which an open and retrieval string or an open spring or a retrieval noose can pass. As the open end of the specimen pouch is provided with a flexible wall of discontinuous serration, and the open and retrieval string or the retrieval noose passes through the slots on the discontinuous serration of the flexible wall of the open end of the specimen pouch, when the open and retrieval string is pulled towards an outer sheath to retrieve the specimen pouch of the invention, the bulk of contracted open end is reduced due to the discontinuous serration of the flexible wall of the open end. It is easy to close the open end of the specimen pouch, and convenient to retrieve the specimen pouch into the outer sheath or extract it through a small incision. The specimen pouch of the invention is simple, convenient, safe and efficient.

## Description

### TECHNICAL FIELD

This invention relates to a kind of surgical instrument used in the retrieval of biological specimens, especially a specimen pouch used in the retrieval of removed biological specimen collected with endoscopic equipment.

### BACKGROUND ART

Endoscopic surgery, celioscope surgery in particular, is a micro-invasive surgery done through a small incision. This way of surgery reduces or even eliminates large incision so that extensive open surgery like the removal of cholecystectomy or myoma of the uterus becomes simple clinical surgery. The recovery phase can be reduced from several weeks to several days; therefore, this technology will receive more and more extensive applications.

However, in the operation of such surgeries, where the removed biological specimen is too big to extract from the small incision, we must enlarge the incision. Thus, it greatly reduces the advantage of endoscopic surgery. Another method is to divide the large biological specimen into several small tissues which are suitable for extraction from the small incision by the surgical instrument. The problem of this method is that fluid leakage or leftover tissue may remain in the body cavity, especially when fluid-filled tissues like cholecyst, cyst, appendix inflammation or malignancy tissues are removed. In these cases, it can easily lead to infection, another syndrome or pervasion of cancer cells, endangering healthy tissues and life security.

In order to solve the difficulty of the endoscopic surgery development, which is restricted by how to extract the large biological specimen through a small incision safely, various kinds of specimen pouches used in the retrieval of biological specimens collected with the endoscopic equipment have been developed by scientists, at home and abroad. The prior art specimen pouch is a soft pouch structure with one end open and the other closed, in which the removed biological specimen can be contained. Some examples of specimen pouch are disclosed in U.S. Pat. No. 5,465,731 to Bell et al., U.S. Pat. No. 5,480,404 to Kammerer et al., U.S. Pat. No. 5,647,372 to Tovey et al., and U.S. Pat. No. 5,971,995 to Rousseau et al. The core of these patents is to first open the open end of the specimen pouch with an open spring, then put the removed biological specimen into the pouch. After that, by starting a retrieval switch, the open spring and the specimen pouch are separated. Then we can close the open end by a retrieval noose and take out the specimen pouch. These different patents are acquired by using different improvements to prevent accidental separation of the open spring and specimen pouch. These products have been applied to clinical surgery effectively. What's more, in the Chinese patent No. ZL01245792.2, Jin Haiming proposed a ballonet specimen pouch. But sometimes the surgeon needs to nip the open end of the specimen pouch with the surgery instrument jaw to bring the removed specimen into the pouch; as a result, risks exist if air leaks out of the ballonet equipment. In the Chinese patent No. ZL01232360.8, Liu Fengru issued a simple-structured specimen pouch, which installed a soft collecting thread at the opening of a soft plastic specimen pouch. Because no open spring or similar functional instruments are placed therein, the opening cannot automatically open. Then we need to open the pouch by using the surgery instruments under the endoscopic equipment, in order to put the removed biological specimen into the specimen pouch. In that case, the inconvenient operation of receiving the biological specimen extends the time of surgery.

It is difficult to close the prior art specimen pouch at the open end due to the rumple resulted from the large bulk of the open end and it is also hard to collect the specimen pouch through the outer sheath or small incisions. Therefore, in the Chinese patent No. ZL0215153.3, S. P. Conlon et al. designed a specimen pouch which can prevent the rumple during its retrieval and eliminate its influence when closing the pouch. Such a specimen pouch employs a flexible wall which is interspersed with soft and rigid pleats in its open end. It can produce centripetal contraction in the course of straining the retrieval noose so that it avoids the influence of the closing of the open end resulting from the anomalistic rumple. Although the influence of the closing of the open end resulting from the anomalistic rumple while straining the retrieval noose has been effectively solved in the above method, bigger retrieval outer sheath or incisions are used to withdraw or take out the frapped open end due to the large bulk of the open end.

In order to overcome the defects, we need to make some improvements to the prior art specimen pouch to provide one with a simpler structure, easier operation, one that is safer and more effective, especially more convenient, to relieve and collect specimens through the outer sheath. At present, no a specimen pouch can meet with these requirements.

### SUMMARY OF THE INVENTION

The easily retrieved biological specimen pouch comprises a flexible wall, an open end and a closed end, and the specimen pouch can receive biological specimens;
A) The flexible wall of the open end of the specimen pouch has discontinuous serration;
B) On the serration, there are slots through which an open and retrieval string or an open spring or a retrieval noose can pass.

The open and retrieval string can be made of the following materials: shape memory alloy wires or shape memory alloy pieces, alloy spring steel or any other materials which can save the changed shape and return to the original or near the original shape when disentangled.

The open spring can be made of the following materials: shape memory alloy wires or shape memory alloy pieces, alloy spring steel or any other materials which can save the changed shape and return to the original or near the original shape when disentangled.

The retrieval noose is made of wires of macromolecule materials, compound materials or metal materials.

The flexible wall of the specimen pouch can be made of soft macromolecule materials or compound materials, and also soft macromolecule materials or the compound materials enhanced by metal net or synthetic fiber, such as the following elastomer or polymer materials: silicon rubber, polyurethane, polyethylene, polypropylene, silicone, ethenoid resin or polytetrafluoroethylene, In addition, it can be made of silicon rubber, polyurethane, polyethylene, polypropylene, silicone, ethenoid resin or polytetrafluoroethylene, which are enhanced by memory alloy fiber net or synthetic fiber net.

The open and retrieval string can be connected to the distant end of the inner sheath, and the specimen pouch can be installed in front of the distant end of the inner sheath and inside the distant end of the outer sheath.

One end of the open and retrieval string can connect with a slipknot or slip block. The noose structure is formed when the other end passes through the slots in the serration of the open end of the specimen pouch and then the slipknot or slip block.

The relative position of the outer sheath and inner sheath can be fixed by an orientation button.

The open end of the specimen pouch can be colored distinctly from the biology specimen observed under the endoscopic equipment.

According to this invention, as the open end of the specimen pouch is provided with a flexible wall of discontinuous serration, and the open and retrieval string or the retrieval noose passes through the slots on the discontinuous serration of the flexible wall of the open end of the specimen pouch, when the open and retrieval string or the retrieved noose is pulled towards the outer sheath to retrieve the specimen pouch of the invention, the bulk of the contracted open end is reduced due to the discontinuous serration of the flexible wall of the open end. It is easy to close the open end of the specimen pouch, and convenient to retrieve the specimen pouch into the outer sheath or extract it through the small incision. The specimen pouch of the invention is simple, convenient, safe and efficient.

### BRIEF DESCRIPTION OF THE DRAWINGS

FIG. 1 is a perspective view illustrating the structure of the easily retrieved biological specimen pouch of this invention.
FIG. 2 is an enlarged, partially sectioned view of part A in FIG. 1.
FIG. 3 is a sectional view along line B-B in FIG. 2.
FIG. 4 is a perspective view illustrating the structure of the easily retrieved biological specimen pouch of this invention with an open and retrieval string using a slipknot noose structure.
FIG. 5 is a perspective view illustrating the structure of the easily retrieved biological specimen pouch of this invention with an open and retrieval string using a slip block noose structure
FIG. 6 is a perspective view illustrating the structure of the easily retrieved biological specimen pouch of this invention with an open spring and an retrieval noose.
FIG. 7 is a perspective view illustrating the structure of the easily retrieved biological specimen pouch of this invention installed inside the inner sheath.
FIG. 8 illustrates the mechanism of receiving the specimen by the easily retrieved biological specimen pouch of this invention.
FIG. 9 illustrates the mechanism of collecting the easily retrieved biological specimen pouch to the outer sheath.

In the above figures, 1 denotes the specimen pouch of this invention; 1-1 denotes the flexible wall of the specimen pouch; 1-2 denotes the open end; 1-3 denotes the closed end; 1-4 denotes the serration of the flexible wall in the open end; 1-5 denotes the slot in the serration of the flexible wall of the open end; 1-6 denotes solder thread; 2 denotes the open and retrieval string; 3 denotes the open spring; 4 denotes the retrieval noose; 5 denotes the inner sheath; 5-1 denotes the distant end of the inner sheath; 5-2 denotes the near end of the inner sheath; 5-3 denotes the handle of the inner sheath; 6 denotes the outer sheath; 6-1 denotes the distant end of the outer sheath; 6-2 denotes the near end of the outer sheath; 6-3 denotes the handle of the outer sheath; 7 denotes the slipknot or slip block; 8 denotes the orientation button; 9 denotes the biological specimen.

### DETAILED DESCRIPTION OF THE INVENTION

### Example 1:

In this experiment, we first get NiTi shape memory alloy whose recovery temperature is 30°C - 33°C, and then make it round above its recovery temperature. Thus, the open and retrieval string 2 of this invention is formed. The open and retrieval string 2 made of the NiTi shape memory alloy can drive the expansion of the open end 1-2 of the specimen pouch; it can also function as frapping the retrieval noose and closing the open end 1-2 of the specimen pouch when collecting the pouch.

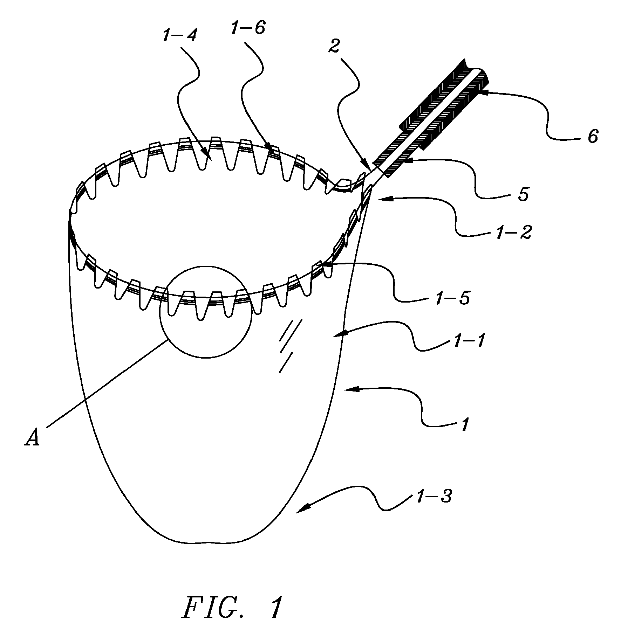

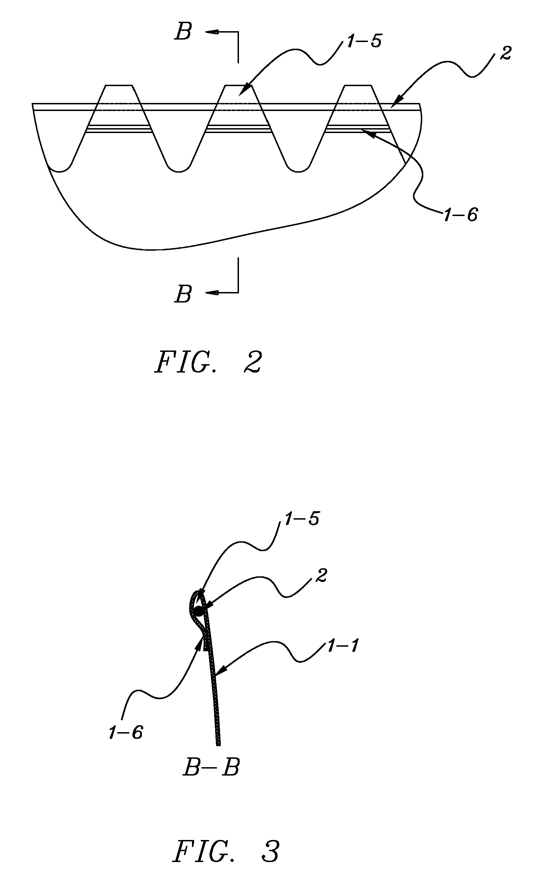

The flexible wall of the specimen pouch 1-1 is made of transparent polyethylene membrane that forms a pocket with one closed end 1-3 and one open end 1-2 after thermoplastic jointing. By the thermoplastic jointing, slots 1-5 are shaped in the open end 1-2. We then cut the open end into hackle shape 1-4, and make the open and retrieval string 2 pass through the slots 1-5 therein to connect with the distant end of the inner sheath. Below the recovery temperature, such as 15°C ~ 20°C, furling the specimen pouch from the close end 1-3 to the open end 1-2, in respect that the open and retrieval string 2 is in a condition whose shape is easily modified, we can easily compress and furl it. Then we put it inside the distant end 6-1 of the outer sheath 6 and in front of the distant end 5-1 of the inner sheath 5, so the easily retrieved biological specimen pouch is obtained. FIG.1, FIG. 2, FIG. 3 and FIG.7 are the references.

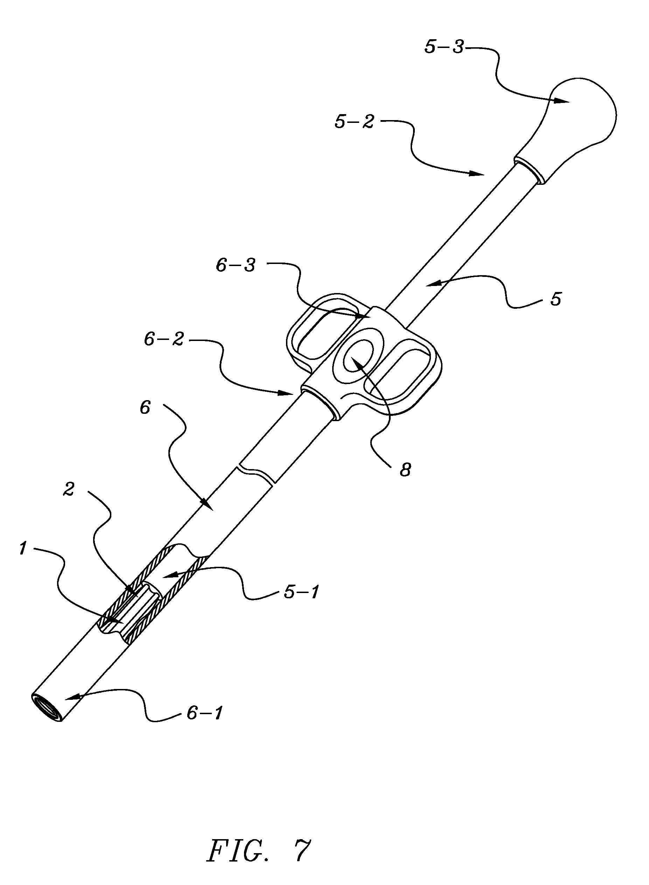

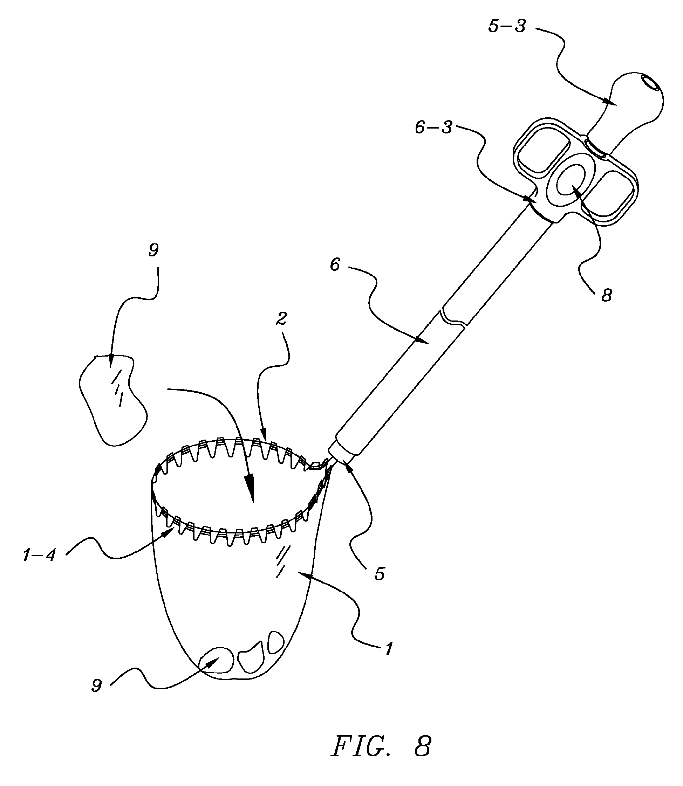

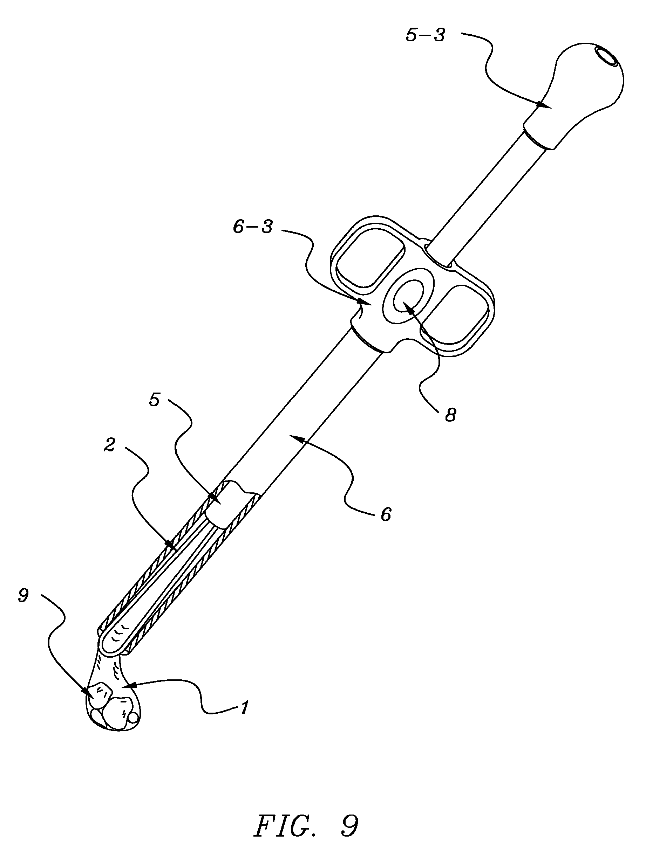

When applied in clinical surgery, we may first insert the outer sheath into the body cavity through small incision, and then pull forth the handle 6-3 of the outer sheath to expose the specimen pouch. With the influence of body heat, the open and retrieval string 2 recovers to the designed shape so that it drives the expansion of the open end 1-2 of the pouch 1. Therefore, the removed biological specimen 9 is brought into the specimen pouch 1 and can be extracted out of the cavity by the rinse sheath after fragmentation by the surgery instrument in the pouch 1. We may then pull forth the handle of the inner sheath 5-3 or push ahead the handle of the outer sheath 6-3, strain the open and retrieval string 2 and close the open end 1-2. Thus, the specimen pouch 1 can be collected to the outer sheath 6 and taken out of the body cavity through the small incision. FIG.7, FIG.8 and FIG.9 are the references.

### Example 2:

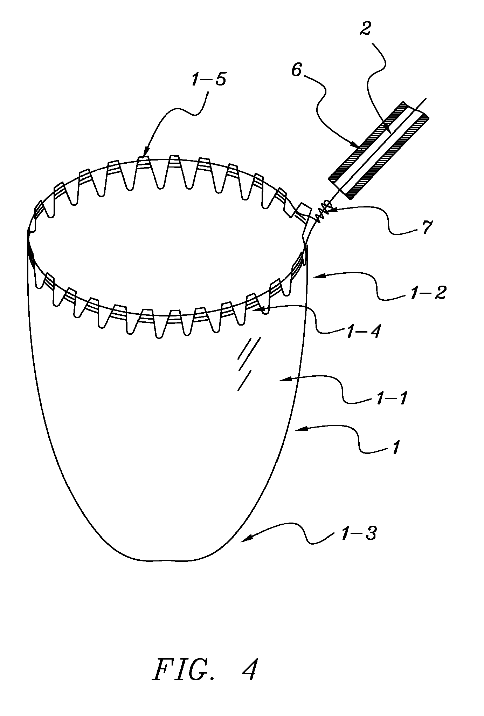

The open and retrieval string 2 is made of a super-elastic metal thread or compound materials thread, which passes through the slots 1-5 in the serration 1-4 of the open end 1-2 in the pouch 1 and forms the slipknot structure on one end. When collecting the pouch 1 by the outer sheath 6, we can pull forth the open and retrieval string 2 through the central bore of the outer sheath 6, then strain the open end 1-2 of the pouch 1. The last step is to pull the pouch with the strained open end to the outer sheath 6. FIG. 4 is the reference.

### Example 3:

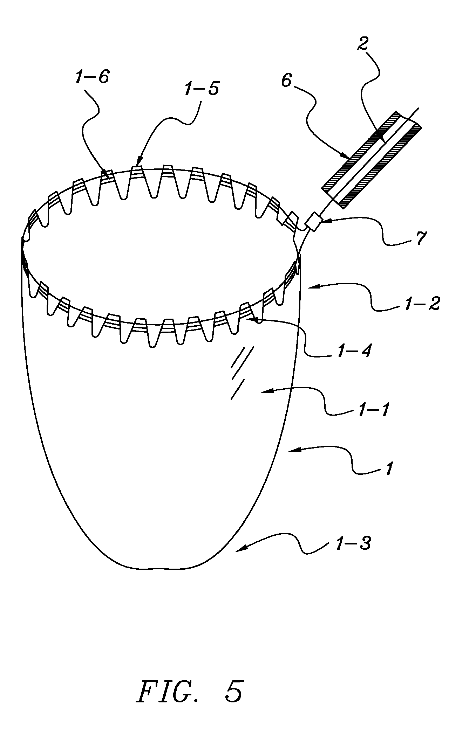

The open and retrieval string 2 is made of NiTi memory alloy wires that are disposed with case-hardened heat treating, while the slip block 7 is made of stainless steel. One end of the open and retrieval string 2 is fixed on the slip block 7 while the other end passes through the slots 1-5 and then through the connect bore of the slip block 7, the noose is formed hereby. When collecting the pouch 1 by the outer sheath 6, we can pull forth the open and retrieval string 2 through the central bore of the outer sheath 6, then strain the open end 1-2 of the pouch 1. The last step is to pull the pouch with the strained open end to the outer sheath 6. FIG. 5 is the reference.

### Example 4:

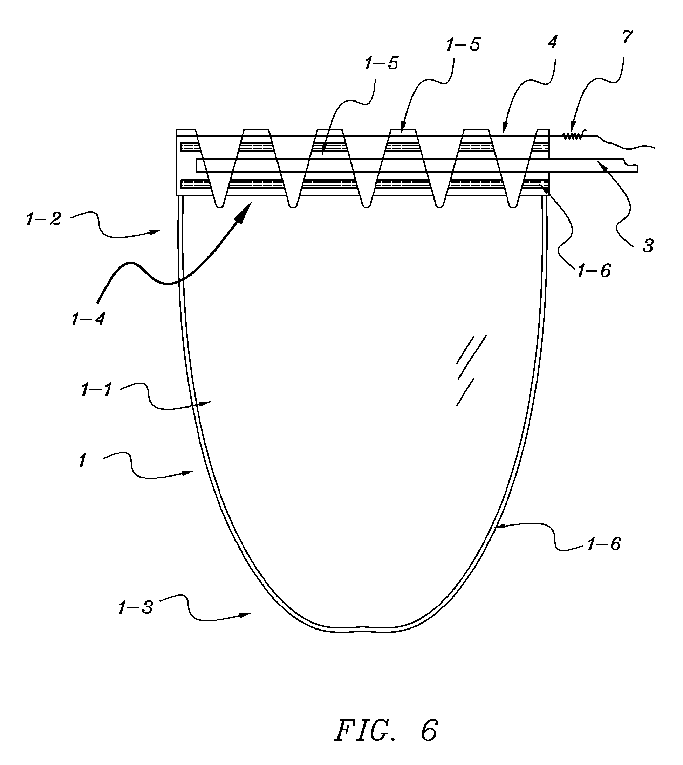

The open spring 3 and the retrieval noose 4 are employed here. After the open spring 3 expands the open end 1-2 in the pouch 1 and receives the specimen, we may withdraw it and strain the retrievable noose 4. Closing the open end 1-2 in the pouch 1, the pouch 1 can be taken out through the small incision after collecting into the outer sheath 6. FIG. 6 is the reference.

Additionally, the flexible wall 1-1 of the specimen pouch can be made of soft macromolecule materials or compound materials. It can also be made of soft macromolecule materials and compound materials enhanced by metal net or synthetic fiber. For examples, the flexible wall is made of the following elastomer or polymer materials: silicon rubber, polyurethane, polyethylene, polypropylene, silicone, ethenoid resin and polytetrafluoroethylene. Moreover, it can be made of silicon rubber, polyurethane, polyethylene, polypropylene, silicone, ethenoid resin or polytetrafluoroethylene, which are enhanced by memory alloy fiber net or synthetic fiber net.

The open and retrieval string 2 or the open spring 3 can be made of the following materials: shape memory alloy wires or pieces, alloy spring steel or any other materials which can save the changed shape and return to the original or near the original shape when disentangled.

The orientation button 8 between the inner sheath 5 and the outer sheath 6 may fix or slack the relative position of the inner sheath 5 and outer sheath 6 in the way of concave-convex assorted structure, screw tighten structure and eccentricity annulus fixation device.

It should be noted that the structure mentioned above in this invention can be replaced by any other structure sharing the same effects, and the examples introduced in this invention are not single structures that can be performed. Although the above examples are presented in this invention, it is to be understood by those who skilled in the art that numerous variations, improvements and substitutes may be effected without departing from the invention. Therefore, it should be define the range of protection according to the scope of the claims of this invention.

## Claims

1. An easily retrieved biological specimen pouch comprising a flexible wall (1-1), an open end (1-2) and a closed end (1-3), and said specimen pouch (1) can receive biological specimen (9) therein;
A) said flexible wall of the open end of the specimen pouch has discontinuous serration (1-4);
B) on said serration (1-4), there are slots (1-5) through which an open and retrieval string (2) or an open spring (3) or a retrieval noose (4) can pass.

2. The biological specimen pouch according to claim 1, wherein said open and retrieval string (2) is made of any materials which can save the changed shape and return to the original or near the original shape when disentangled.

3. The biological specimen pouch according to claim 2, wherein said open and retrieval string (2) is made of the following materials: shape memory alloy wires or pieces or alloy spring steel.

4. The biological specimen pouch according to claim 1, wherein said open spring (3) is made of any materials which can save the changed shape and return to the original or near the original shape when disentangled.

5. The biological specimen pouch according to claim 4, wherein said open spring (3) is made of the following materials: shape memory alloy wires, shape memory alloy pieces and alloy spring steel.

6. The biological specimen pouch according to claim 1, wherein said retrieval noose (4) is made of wires of macromolecule materials, compound materials or metal materials.

7. The biological specimen pouch according to claim 1, wherein said flexible wall (1-1) of the specimen pouch is made of soft macromolecule materials or compound materials.

8. The biological specimen pouch according to claim 1, wherein said flexible wall (1-1) of the specimen pouch is made of soft macromolecule materials or compound materials which are enhanced by metal net or synthetic fiber.

9. The biological specimen pouch according to claim 1, wherein said flexible wall (1-1) of the specimen pouch is made of soft macromolecule materials or compound materials which are enhanced by memory alloy fiber net or synthetic fiber net.

10. The biological specimen pouch as claimed in any one of claims 7 to 9, wherein said soft macromolecule materials are selected from the following elastomer or polymer materials: silicon rubber, polyurethane, polyethylene, polypropylene, silicone, ethenoid resin and polytetrafluoroethylene.

11. The biological specimen pouch according to claim 1, wherein said open and retrieval string (2) is connected to a distant end (5-1) of an inner sheath (5), and the specimen pouch (1) is installed in front of the distant end (5-1) of the inner sheath and inside a distant end (6-1) of an outer sheath (6).

12. The biological specimen pouch according to claim 1, wherein one end of said open and retrieval string (2) is connected with a slipknot or slip block (7), a noose structure is formed when the other end passes through the slots (1-5) in the serration (1-4) of the open end in the specimen pouch and then the slipknot or slip block (7).

13. The biological specimen pouch according to claim 1, wherein the relative position of the outer sheath (5) and inner sheath (6) is fixed by an orientation button (8).

14. The biological specimen pouch according to claim 1, wherein said open end (1-2) of the specimen pouch is colored distinctly from the biological specimen (9) observed under endoscopic equipment.
